(19) Europäisches Patentamt
European Patent Office
Office européen des brevets

(11) Numéro de publication : **0 006 061**
**B1**

(12) **FASCICULE DE BREVET EUROPÉEN**

(45) Date de publication du fascicule du brevet : **24.02.82**

(51) Int. Cl.$^3$ : **A 61 K 31/11**, A 61 K 31/045, A 61 K 31/19

(21) Numéro de dépôt : **79400340.0**

(22) Date de dépôt : **29.05.79**

(54) **Médicament constitué par un dérivé de cyclopentyl-aldéhyde.**

(30) Priorité : **03.06.78 GB 2631678**

(43) Date de publication de la demande : **12.12.79 (Bulletin 79/25)**

(45) Mention de la délivrance du brevet : **24.02.82 Bulletin 82/08**

(84) Etats contractants désignés : **AT DE NL SE**

(56) Documents cités :
**CHEMICAL ABSTRACTS, Vol. 88, 1978 réf. : 11723f, page 286 COLUMBUS, OHIO (US).**
**Austr. J. Chem, (1976), *29*, 1375-1381**

(73) Titulaire : **INSTITUT DE RECHERCHES CHIMIQUES ET BIOLOGIQUES APPLIQUEES (I.R.C.E.B.A.) Société à responsabilité limitée dite: 28 rue de Téhéran F-75008 Paris (FR)**

(72) Inventeur : **Debat, Jacques**
**3 rue du Pierrier**
**F-92210 Saint Cloud (FR)**
Inventeur : **Lemoine, Jean**
**7 Sente de la Portaille**
**F-92380 Garches (FR)**
Inventeur : **Riffaud, Jean-Pierre**
**48 Rue Albert Joly**
**F-78000 Versailles (FR)**

(74) Mandataire : **Clisci, Serge et al**
**CABINET BEAU DE LOMENIE 55 rue d'Amsterdam**
**F-75008 Paris (FR)**

**EP 0 006 061 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Médicament constitué par un dérivé de cyclopentyl-aldéhyde

La présente invention a trait à un nouveau médicament qui est constitué par un dérivé appartenant à la famille des cyclopentyl-aldéhydes.

On sait que BEAUPIN et al., Rivista Italiana Essenze, Profumi, Piante Officinali, Aromi, Saponi, Cosmetici, Aerosol *59* (No 8), 369-373 (1977) et PAGNONI et al., Austr. J. Chem., *29*, 1 373-1 381 (1976) ont décrit un grand nombre de substances chimiques isolées de *Teucrium marum* sans avoir étudié leurs éventuelles propriétés pharmacologiques. Ils ont en particulier décrit, en tant que produits chimiques, les isomères dolichodials de formule I ci-après (où X = Y = CHO, Z = $CH_3$ et A = >C = $CH_2$) et des lactones structurellement différentes des dolichodials.

On vient de trouver de façon surprenante que les dérivés de cyclopentyl-aldéhyde sont utiles en thérapeutique en raison de leurs propriétés antianaphylactiques.

Plus précisément, l'invention a trait à un dérivé cyclopentyl-aldéhyde utile en thérapeutique qui est choisi parmi l'ensemble constitué par :

a) les composés de formule générale

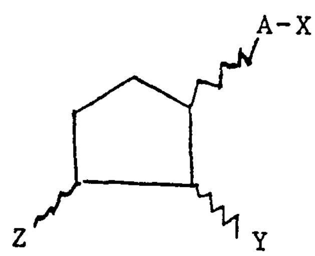

(I)

dans laquelle X et Y, qui peuvent être identiques ou différents, représentent chacun CHO, $CH_2OH$, ou COOH ; Z représente un groupe alkyle en $C_1$-$C_3$ ; A représente —$CH_2$—, >C = $CH_2$ ou >CH($CH_3$) ; et, leurs isomères géométriques.

Les composés de formule I sont des substances connues qui peuvent être préparées soit par synthèse, soit par extraction à partir d'une plante appartenant à la famille des *Labiacées* telles que les espèces *Teucrium marum, Teucrium polium* et *Teucrium montanum.* Par exemple, ils peuvent être extraits du *Teucrium marum* selon le procédé décrit par PAGNONI et al., dans l'article précité.

Les dérivés de cyclopentyl-aldéhyde de formule I sont (i) actifs en tant qu'agents bactériostatiques, bactéricides, antispasmodiques, antianaphylactiques et antiinflammatoires, et (ii) utiles en particulier dans le traitement des êtres humains et des animaux à sang chaud souffrant de maladies infectieuses provoquées par des germes Gram (+) et Gram (−), et des allergies telles que le rhum des foins et l'asthme.

Par ailleurs, les dérivés de cyclopentyl-aldéhyde de formule I sont plus intéressants, du point de vue pharmaceutique, que les extraits de *Teucrium marum* selon le brevet américain No 4 151 278.

Parmi les composés inclus dans la définition de la formule I, on peut notamment citer les composés suivants :

— Ex. 1 : α-(2-formyl-3-méthyl-cyclopentyl)-acétaldéhyde (X = Y = CHO ; A = $CH_2$ ; Z = $CH_3$),

— Ex. 2 : 2-(2-hydroxyméthyl-3-méthyl-cyclopentyl)-éthanol (X = Y = $CH_2OH$ ; A = $CH_2$ ; Z = $CH_3$),

— Ex. 3 : acide α-(2-carboxy-3-méthyl-cyclopentyl)-acétique (X = Y = COOH ; A = $CH_2$ ; Z = $CH_3$),

— Ex. 4 : α-(2-formyl-3-méthyl-cyclopentyl)-acrylaldéhyde (X = Y = CHO ; A = C = $CH_2$ ; Z = $CH_3$),

— Ex. 5 : 2-(2-hydroxyméthyl-3-méthyl-cyclopentyl)-2-propène-1-ol (X = Y = $CH_2OH$ ; A = C = $CH_2$ ; Z = $CH_3$),

— Ex. 6 : acide α-(2-carboxy-3-méthyl-cyclopentyl)-acrylique (X = Y = COOH ; A = C = $CH_2$ ; Z = $CH_3$), et

— Ex. 7 : 2-(2-hydroxyméthyl-3-méthyl-cyclopentyl)-propan-1-ol (X = Y = $CH_2OH$ ; A = CH($CH_3$) ; Z = $CH_3$),

qui correspondent tous à la formule suivante

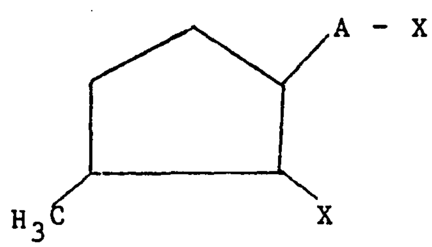

(II)

(dans laquelle A et X sont définis comme ci-dessus) et présentent principalement deux isomères :

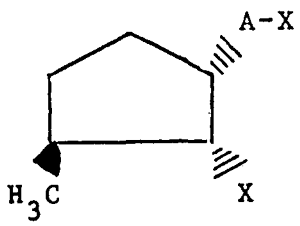

(A)

« cis-trans »

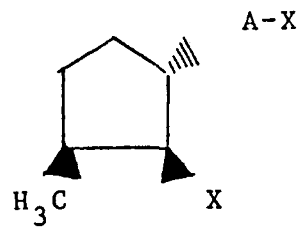

(B)

« trans-cis »

l'isomère « cis-trans » étant, d'une façon générale, plus actif que l'isomère « trans-cis ».

Les composés préférés sont ceux des exemples 1 et 4. Le plus intéressant, du point de vue pharmaceutique, est le composé de l'exemple 4.

Le mode préféré pour préparer les composés « acryle » (où A est $>C = CH_2$) consiste à extraire le α-(2-formyl-3-méthyl-cyclopentyl)-acrylaldéhyde à partir d'une espèce de *Teucrium* tels que les *Teucrium marum, Teucrium polium, Teucrium montanum,* d'une part, et à transformer CHO en $CH_2OH$ par réduction ou en COOH par oxydation, d'autre part. Les composés dans lesquels A est $>CH(CH_3)$ tel que le produit de l'exemple 7, sont obtenus par hydrogénation catalytique. Les composés dans lesquels A est $CH_2$ sont préparés en particulier par synthèse selon une méthode connue en soi.

PREPARATION I

Obtention de α-(2-formyl-3-méthyl-cyclopentyl)-acrylaldéhyde (Exemple 4)

1 210 g de *Teucrium polium* (plante entière) séchés et broyés sont extraits dans une colonne avec successivement 7,35 litres d'hexane et 7,25 litres de chloroforme. L'extrait chloroformique est évaporé à sec sous vide et le résidu ainsi obtenu (45 g) est entraîné à la vapeur d'eau. Le distillat est saturé avec du chlorure de sodium, puis extrait au chloroforme après séchage sur $Na_2SO_4$ ; le chloroforme est éliminé par distillation sous pression réduite (0,3 mm Hg) pour donner 7,3 g (rendement 0,6 %) d'une huile jaune ($n_D^{25\,°C}$ = 1,484 2) comprenant

89 % en poids de

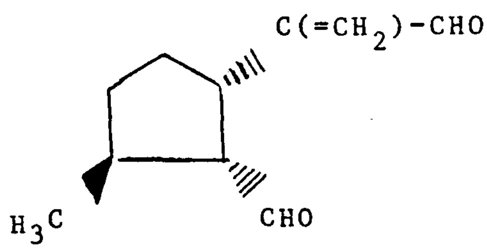

(« cis-trans »)

et

11 % en poids de

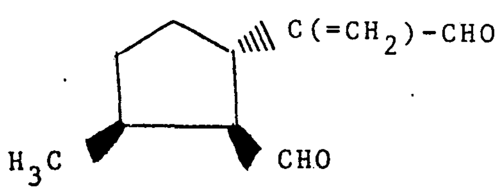

(« trans-cis »)

PREPARATION II

Obtention de α-(2-formyl-3-méthyl-cyclopentyl)-acrylaldéhyde (exemple 4)

25 kg de *Teucrium marum* (plante entière) séchés et broyés sont extraits par lixiviation dans une

colonne en verre (hauteur : 2 m ; ∅ : 0,225 m) remplie de solvant au moyen d'une pompe ayant un débit de 10 l/h. Deux solvants sont utilisés successivement : hexane (170 litres) puis chloroforme (170 litres). L'extrait à l'hexane est écarté. L'extrait chloroformique est évaporé à sec sous vide pour donner un résidu huileux brun (640 g) qui est entraîné à la vapeur. Le distillat est saturé avec NaCl puis extrait au chloroforme. La phase chloroformique est séchée sur $NaSO_4$, le solvant est évaporé et l'huile restante est distillée (sous 1 mm Hg à 60-78 °C) pour donner 42 g d'une huile jaune ($n_D^{25\,°C}$ = 1,484 2 qui comprend :

— 87 % en poids d'isomère « cis-trans »
— 13 % en poids d'isomère « trans-cis »

PREPARATION III

Obtention de 2-(2-hydroxyméthyl-3-méthyl-cyclopentyl)-2-propène-1-ol (exemple 5)

Par réduction de l'isomère « cis-trans » de l'exemple 4 (1,4 g) en solution dans $CH_3OH$ (25 ml) avec $NaBH_4$ (1,7 g), l'isomère « cis-trans » de l'exemple 5 (1,19 g) est obtenu sous la forme d'une huile. Selon la même technique, on prépare l'isomère « trans-cis » de l'exemple 5 à partir de l'isomère « trans-cis » de l'exemple 4.

PREPARATION IV

Obtention de 2-(2-hydroxyméthyl-3-méthyl-cyclopentyl)-propan-1-ol (exemple 7)

Les isomères « cis-trans » et « trans-cis » de l'exemple 7 sont obtenus par hydrogénation sur $PtO_2$ des isomères correspondant du produit de l'exemple 5 selon la technique décrite par PAGNONI et al.

Les essais pharmaceutiques qui ont été mis en œuvre avec les produits des exemples 1 et 4, ont été résumés ci-après :

1. essais sur l'exemple 1

a) activité bactériostatique

L'α-(2-formyl-3-méthyl-cyclopentyl)-acétaldéhyde inhibe les bactéries Gram (+) et Gram (−). Les CMI (concentration minimale inhibitrice) sont les suivantes :

0,75 mg/ml sur *Staphylococcus aureus* Londres,
1,2 mg/ml sur *Escherichia coli*, et
1,5 mg/ml sur *Proteus*

b) activité antispasmodique

Les DE-50 du produit de l'exemple 1 ont été déterminés

(i) in vitro sur iléon isolé de cobaye vis à vis de l'histamine :

DE − 50 = 50 γ/ml ;

(ii) in vivo sur l'animal vivant selon la méthode de KONZETT & ROESLLER :

DE − 50 = 2,6 mg/kg par administration i.v.

c) activité antianaphylactique :

Des cobayes mâles sont sensibilisés par deux injections de 0,5 ml d'une solution d'albumine à 2 g/l, à deux jours d'intervalle. Quatre semaines après, le choc anaphylactique mortel est provoqué par injection i.v. de 0,2 ml d'une solution aqueuse d'ovalbumine à 2 g/l. Les produits à tester sont administrés 30 mn avant (administration i.p.) ou en même temps (administration i.v.) que l'injection provoquant ledit choc anaphylactique. Les résultats sont donnés dans le tableau I pour le produit de l'exemple 1 et la Prométhazine en tant que produit de référence :

TABLEAU I

| Composé (dose) | Mortalité % | Protection % |
|---|---|---|
| Témoins | 80 % | 20 % |
| Prométhazine (20 mg/kg i.p.) | 0 % | 100 % |
| Exemple 1 (10 mg/kg i.p.) | 50 % | 50 % |
| Exemple 1 (2 mg/kg i.v.) | 0 % | 100 % |

2. essais avec le produit de l'exemple 4 (constitué par un mélange de 87 % en poids d'isomère « cis-trans » et 13 % en poids d'isomère « trans-cis »).

a) activité bactériostatique

Le produit de l'exemple 4 inhibe les bactéries Gram (+) et Gram (−). Ses CMI sont les suivantes :

1,5 mg/ml sur *Staphylococcus aureus*, Londres
1 mg/ml sur *Escherichia coli* (souche 548)
1,75 mg/ml sur *Proteus vulgaris* (souche 1 557)
2,5 mg/ml sur *Klebsiella pneumoniae* (souche 433),
1 mg/ml sur *Salmonella typhi murium* (souche IP),
1,5 mg/ml sur *Staphylococcus aureus* (souche 634),
1,25 mg/ml sur *Streptococcus* (souche 1 178)
1,25 mg/ml sur *Bacillus substilis*,

(les souches 548, 1 557, 433, 634 et 1 178 étant celles du catalogue de la collection du « Centre International de Distribution de Souches et d'Information sur les Types Microbiens » de Lausanne, et la souche « IP » provenant de l'Institut Pasteur de Paris.

b) activité bactéricide

Le produit de l'exemple 4 détruit les bactéries Gram (+) et Gram (−). Ses CMB (concentration minimale bactéricide) déterminées sur milieu solide (quand moins de 0,01 % de germes survivent) sont :

1,75 mg/ml sur *Staphylococcus aureus* Londres
1,5 mg/ml sur *Escherichia coli* (souche 548)
2 mg/ml sur *Proteus vulgaris* (souche 1 557)
3 mg/ml sur *Klebsiella pneumoniae* (souche 433),
1,25 mg/ml sur *Salmonella typhi murium* (souche I.P.)
1,75 mg/ml sur *Staphylococcus aureus* (souche 634)
2 mg/ml sur *Streptococcus* (souche 1 178)
2,5 mg/ml sur *Bacillus substilis*.

c) activité bronchospatique

Des cobayes mâles ou femelles [20 animaux (pesant 300-500 g) par dose et par produit à tester] sont exposés à un aérosol de l'exemple 4 (50 g/l en solution eau-éthanol) ou de Prométhazine (produit de référence) pendant 10 mn.

Les animaux sont ensuite exposés individuellement à un aérosol d'histamine (3 g/l d'histamine dans un milieu aqueux contenant 200 g/l de glycérine).

Le critère choisi est le temps écoulé entre le début de l'exposition à l'histamine et l'apnée, accompagnée de chute de l'animal.

Le lot témoins ne reçoit que l'aérosol d'histamine. Les résultats ont été donnés dans le tableau II et montrent que le produit de l'exemple 4 possède un effet bronchodilatateur qui s'oppose à l'effet bronchoconstricteur de l'histamine :

TABLEAU II

| Produit (dose) | Apparition de l'apnée (en secondes) | Variation vis-à-vis des témoins |
|---|---|---|
| Témoins | 108 ± 18 | — |
| Prométhazine 0,1 mg/kg | > 600 | — |
| Exemple 4 (1 mg/kg) | 130 ± 23 (*) | + 20 % |
| Exemple 4 (10 mg/kg) | 169 ± 32 (*) | + 56 % |

(*) Statistiquement significatif (P < 0,05).

d) activité antianaphylactique

in vitro

Des cobayes mâles sont sensibilisés par une injection i.p. de 100 mg/kg d'ovalbumine dans un volume égal d'adjuvant de Freund. Quatre semaines après, les animaux sont sacrifiés, on recueille chaque iléon qui est placé à 33 °C dans un bain de Thyrode. Une contraction est provoquée par addition d'une dose de 200 μg d'albumine au liquide de survie. L'amplitude des contractions des témoins est comparée à l'amplitude des contractions après mise en contact du produit de l'exemple 4 pendant 2 mn.

Les résultats donnés dans le tableau III montrent que le produit de l'exemple 4 réduit l'amplitude des contractions de l'iléon isolé de cobaye, cette réduction étant statistiquement significative.

in vivo

Des cobayes (mâles ou femelles) sont sensibilisés à l'ovalbumine quatre semaines avant le début de l'expérience. Chaque animal est exposé à un aérosol d'ovalbumine (obtenu à partir d'un milieu aqueux contenant 50 g/l d'ovalbumine). Le choc anaphylactique se manifeste par l'apparition d'une dyspnée intense. Les temps d'apparition de la dyspnée sont comparés entre lot témoin et lots préalablement traités avec un aérosol du produit de l'exemple 4 (50 g/l dans une solution eau-éthanol). Les résultats donnés dans le tableau IV, montrent que le produit de l'exemple 4 diminue le temps d'apparition des troubles respiratoires provoqués par l'aérosol d'ovalbumine.

TABLEAU III

| Produit (dose) | Nombre d'animaux | Amplitude des contractions | Variation de l'amplitude par rapport aux animaux témoins |
|---|---|---|---|
| Témoins | 20 | 147 ± 32 | — |
| Exemple 4 (5 μg/ml) | 20 | 91 ± 37 (*) | – 38 % |
| Exemple 4 (10 μg/ml) | 20 | 52 ± 16 (*) | – 65 % |

(*) Statistiquement significatif ($p < 0,05$).

TABLEAU IV

| Produit (dose) | Nombre d'animaux | Dyspnée<br>Temps d'apparition (secondes) | Variation du temps d'apparition par rapport aux témoins |
|---|---|---|---|
| Témoins | 20 | 94 ± 39 | — |
| Exemple 4 (1 mg/kg) | 20 | 120 ± 52 | + 28 % |
| Exemple 4 (10 mg/kg) | 20 | 130 ± 54 | + 38 % |

e) activité anti-inflammatoire

L'activité anti-inflammatoire de l'exemple 4 a été étudiée chez le rat femelle (de poids 100 g) selon le test de l'œdème à la carragéenine. Les résultats sont donnés dans le tableau IV où l'Indométacine est utilisée comme produit de référence :

TABLEAU V

| Produit | Nombre d'animaux | Dose (par rat) | Inhibition par rapport aux témoins |
|---|---|---|---|
| Exemple 4 | 10 | 10 μg | 26 % |
| Exemple 4 | 10 | 50 μg | 25 % |
| Exemple 4 | 10 | 200 μg | 41 % |
| Indométacine | 10 | 200 μg | 57 % |

f) activité antispasmodique

L'activité antispasmodique du produit de l'exemple 4 a été étudiée sur le duodénum isolé de rat vis-à-vis de l'acétylcholine et du chlorure de baryum. Les DE-50 du produit de l'exemple 4 sont :

(i) 13 μg/ml vis-à-vis de l'acétylcholine (0,2 μg/ml), et

(ii) 10 μg/ml vis-à-vis de $BaCl_2$ (0,3 μg/ml).

g) toxicité

Les DL-50 du poids de l'exemple 4 chez la souris sont les suivantes :

DL-50 i.p. = 18 ± 3 mg/kg

DL-50 i.v. = 20 ± 3 mg/kg

DL-50 p.o. = 282 ± 3 mg/kg

DL-50 s.c. > 1 000 mg/kg

DL-50 aérosol = 22 ± 3 mg/kg

Selon l'invention, on préconise une composition thérapeutique comprenant, en association avec un excipient physiologiquement acceptable, une quantité pharmaceutiquement efficace d'un dérivé cyclopentyl-aldéhyde de formule I.

Une telle composition peut être administrée oralement sous forme de comprimés, dragées, sirop et ampoules buvables, par injection et sous forme d'aérosol.

Selon le mode préféré de mise en œuvre de l'invention, on administre aux êtres humains et aux animaux à sang chaud, le composé de l'exemple 4 ou son isomère « cis-trans », de préférence sous la forme d'aérosol (en solution dans le carbitol ou en suspension dans le fréon ou un mélange fréon-alcool).

De façon avantageuse, la composition aérosol renfermera 0,1 à 10 % du produit de l'exemple 4 ou de son isomère « cis-trans ».

Le produit de l'exemple 4 et son isomère « cis-trans » peuvent également être administrés par voie orale sous la forme de capsules molles (l'ingrédient actif étant en solution huileuse), de gélules (l'ingrédient actif étant associé à un excipient visqueux ou solide), l'excipient préféré pour les gélules étant notamment un mélange de glycérides oléiques polyoxyéthylénés obtenus par alcoolyse d'huile végétale naturelle et qui sont vendus sous le nom de LABRAFIL.

**Revendications**

1. Médicament utile en thérapeutique dans le traitement des maladies infectieuses, des algies, des inflammations et des allergies notamment le rhume des foins, constitué par un dérivé de cyclopentyl-aldéhyde caractérisé en ce que l'ingrédient actif est choisi parmi l'ensemble constitué par :

a) les dérivés de cyclopentyl-aldéhyde de formule générale

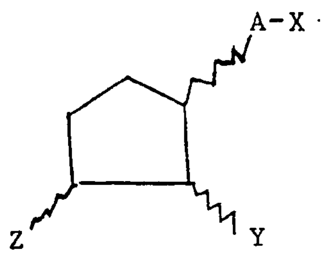

(I)

dans laquelle X et Y, identiques ou différents représentent chacun CHO, $CH_2OH$, ou COOH ; Z représente un groupe alkyle en $C_1$-$C_3$ ; A représente $-CH_2-$, $>C = CH_2$ et $>CH(CH_3)$ ; et

b) leurs isomères géométriques.

2. Médicament selon la revendication 1, caractérisé en ce que X = Y = CHO, $CH_2OH$ et Z = $CH_3$.

3. Médicament selon la revendication 1, caractérisé en ce que l'isomère géométrique est choisi parmi l'ensemble constitué par :

a) les isomères « cis-trans » de formule

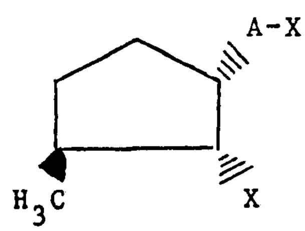

et,

b) les isomères « trans-cis » de formule

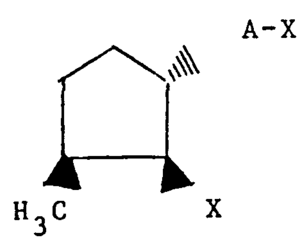

où A et X sont définis comme indiqué dans la revendication 1.

4. Médicament selon la revendication 3, caractérisé en ce que X est CHO et A est $\gt C = CH_2$.

**Claims**

1. A drug useful in therapy in the treatment of infectious diseases, algiae, inflammations and allergies, and in particular hay fever, constituted by a cyclopentylaldehyde derivative, characterised in that the active ingredient is selected form the group consisting of :

a) cyclopentyl-aldehyde derivatives of the general formula

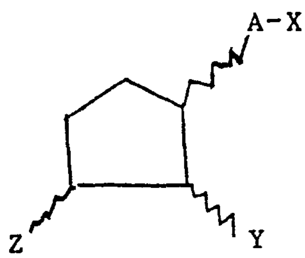

(I)

wherein X and Y, which can be the same or different, represent CHO, $CH_2OH$ or COOH ; Z represents a $C_1$-$C_3$ alkyl group, and A represents $-CH_2-$, $\gt C = CH_2$ and $\gt CH(CH_3)$ ; and

b) their geometrical isomers.

2. A drug according to claim 1, characterised in that X = Y = CHO, $CH_2OH$ and Z = $CH_3$.

3. A drug according to claim 1, characterised in that the geometrical isomer is selected from the group consisting of :

a) « cis-trans » isomers of the formula

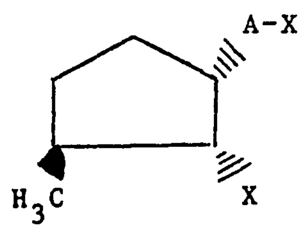

and,

b) « trans-cis » isomers of the formula

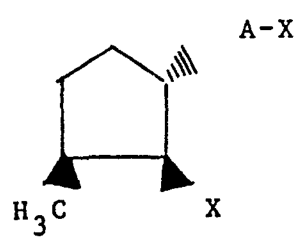

wherein A and X are defined as indicated in claim 1.

4. A drug according to claim 3, characterised in that X is CHO and A is $\gt C = CH_2$.

**Ansprüche**

1. Arzneimittel, das für die Behandlung von Infektionskrankheiten, Algesien, Entzündungen und Allergien, besonders Heuschnupfen, vorteilhaft ist und aus einem Derivat von Cyclopentyl-aldehyd besteht, dadurch gekennzeichnet, dass der aktive Ingrediens aus der Gruppe, die aus

a) den Derivaten von Cyclopentyl-aldehyd der allgemeinen Formel

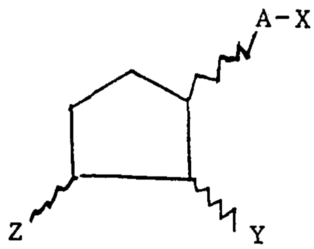

(I)

worin X und Y, die identisch oder unterschiedlich sein können, CHO, $CH_2OH$ oder COOH bedeuten ; Z eine $C_1$-$C_3$ Alkylgruppe bedeutet ; und A $-CH_2-$, $>C = CH_2$ oder $>CH\ (CH_3)$ bedeutet, und

b) deren geometrischen Isomeren besteht, gewählt wird.

2. Arzneimittel nach Anspruch 1, dadurch gekennzeichnet, dass X = Y = CHO oder $CH_2OH$ und Z = $CH_3$.

3. Arzneimittel nach Anspruch 1, dadurch gekennzeichnet, dass das geometrische Isomer aus der Gruppe, die aus

a) den « cis-trans » Isomeren der Formel

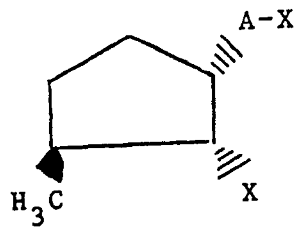

und

b) den « trans-cis » Isomeren der Formel

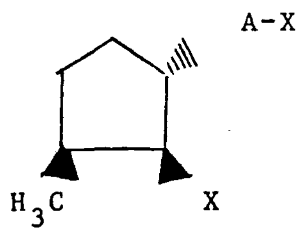

worin A und X wie in Anspruch 1 angegeben definiert sind besteht, gewählt wird.

4. Arzneimittel nach Anspruch 3, dadurch gekennzeichnet, dass X die Gruppe CHO und A die Gruppe $>C = CH_2$ bedeuten.